(19) Europäisches Patentamt
European Patent Office
Office européen des brevets

(11) EP 3 549 671 A1

(12) EUROPÄISCHE PATENTANMELDUNG

(43) Veröffentlichungstag:
**09.10.2019 Patentblatt 2019/41**

(21) Anmeldenummer: **19162180.4**

(22) Anmeldetag: **12.03.2019**

(51) Int Cl.:
***B01J 29/04*** *(2006.01)* ***B01J 37/08*** *(2006.01)*
***B01J 37/03*** *(2006.01)* ***B01J 37/02*** *(2006.01)*
***B01J 35/00*** *(2006.01)* ***B01J 35/10*** *(2006.01)*
***C01B 39/04*** *(2006.01)* ***C07C 2/10*** *(2006.01)*
*B01J 21/12 (2006.01)* *B01J 23/755 (2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **14.03.2018 EP 18161755**

(71) Anmelder: **Evonik Degussa GmbH
45128 Essen (DE)**

(72) Erfinder:
- **Reschetilowski, Wladimir
01445 Radebeul (DE)**
- **Alscher, Felix
01187 Dresden (DE)**
- **Frenzel, Henrik
01099 Dresden (DE)**
- **Borovinskaya, Ekaterina
01796 Pirna (DE)**
- **Breitkopf, Cornelia
01097 Dresden (DE)**
- **Nadolny, Fabian
59821 Arnsberg (DE)**
- **Stochniol, Guido
45721 Haltern am See (DE)**
- **Peitz, Stephan
45739 Oer-Erkenschwick (DE)**
- **Franke, Robert
45772 Marl (DE)**

(74) Vertreter: **Evonik Patent Association
c/o Evonik Industries AG
IP Management
Bau 1042A/PB 15
Paul-Baumann-Straße 1
45772 Marl (DE)**

# (54) VERFAHREN ZUR HERSTELLUNG EINES STRUKTURSELEKTIVEN UND LANGZEITSTABILEN OLIGOMERISIERUNGSKATALYSATORS AUF BASIS VON NICKEL-ALUMOSILICAT DURCH FÄLLUNG

(57) Gegenstand der vorliegenden Erfindung ist ein Verfahren zur Herstellung eines Oligomerisierungskatalysators auf Basis von Nickel-Alumosilicat, das in der heterogenkatalysierten Oligomerisierung von C3- bis C6-Olefinen oder darauf basierenden olefinhaltigen Einsatzgemsichen über eine hohe Aktivität und Selektivität bei ausreichender Standzeit verfügt.

EP 3 549 671 A1

## Beschreibung

Die vorliegende Erfindung betrifft ein Verfahren zur Herstellung eines Nickel-Alumosilicats, das sich vorteilhaft als Oligomerisierungskatalysator zur Oligomerisierung von C3- bis C6-Olefinen sowie deren Gemischen einsetzen lässt. Ein weiterer Gegenstand der vorliegenden Erfindung ist die Verwendung des Oligomerisierungskatalysators in einem Verfahren zur Oligomerisierung von C3-bis C6-Olefinen.

Generell versteht man unter der Oligomerisierung die Reaktion von ungesättigten Kohlenwasserstoffen untereinander, wobei entsprechend längerkettige Kohlenwasserstoffe, die so genannten Oligomere, entstehen. So lässt sich beispielsweise durch die Oligomerisierung von zwei Olefinen mit jeweils drei Kohlenstoffatomen ein Olefin mit sechs Kohlenstoffatomen (Hexen) aufbauen. Die Oligomerisierung von zwei Molekülen miteinander wird auch Dimerisierung, die Oligomerisierung von drei Molekülen miteinander Trimerisierung usw. genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase mit einem gelösten Katalysator oder heterogen an einem festen Katalysator bzw. mit Katalysatoren in einem zweiphasigen Reaktionssystem durchgeführt.

Bei den heterogen katalysierten Verfahren ist die Oligomerisierung an sauren Oligomerisierungskatalysatoren lange bekannt. Technisch werden z. B. acide Zeolithe oder Phosphorsäure auf einem Support eingesetzt. Hierbei werden Isomerengemische von vorwiegend verzweigten Oligomeren erhalten. Für die nicht-saure, heterogen katalysierte Oligomerisierung von Olefinen, bei der hauptsächlich lineare bzw. leicht verzweigte Dimere entstehen, werden in der Technik am häufigsten Nickelverbindungen auf Supportmaterialien eingesetzt. So wird in der WO 95/14647 A1 als Katalysator für die Olefinoligomerisieurng ein Nickelkatalysator mit einem Supportmaterial, das aus den Komponenten Titanoxid und/oder Zirkoniumoxid, Siliciumdioxid und gegebenenfalls Aluminiumoxid besteht, beschrieben. In Gegenwart dieses Katalysators werden Gemische linearer Butene mit einer Selektivität von unter 75 % zu C8-Olefinen oligomerisiert.

Für die Herstellung von Oligomerisierungskatalysatoren sind verschiedene Verfahren aus dem Stand der Technik bekannt. Die WO 95/14647 A1 offenbart beispielsweise ein Verfahren zur Herstellung eines Oligomerisierungskatalysators, bei dem die einzelnen Komponenten aus einer Lösung ausgefällt werden. Die daraus hergestellten Katalysatormaterialien zeigen jedoch erst bei erhöhten Temperaturen und erhöhten Drücken akzeptable Umsätze im Hinblick auf die Bildung von n-Oktenen.

Der vorliegenden Erfindung lag die Aufgabe zugrunde ein neuartiges Verfahren bereitzustellen, mit dem ein Oligomerisierungskatalysator mit verbesserten Eigenschaften einfach hergestellt werden kann und bei dessen Einsatz in der Oligomerisierung von C3- bis C6-Olefinen gute Umsätze und Selektivitäten unter gemäßigten Bedingungen erzielt werden können, wobei kein negativer Effekt auf die Standzeit des Katalysators und die mechanischen Eigenschaften wie Festigkeit erfolgt.

Es hat sich überraschend gezeigt, dass die zugrundeliegende Aufgabe mit einem Verfahren gelöst werden konnte, bei dem der Oligomerisierungskatalysator auf Basis von Nickel-Alumosilicat aus einer Lösung, die mindestens zwei Template enthält, ausgefällt wird. Ein so hergestellter Katalysator führt bei seinem Einsatz in der Oligomerisierung, insbesondere der Oligomerisierung von kurzkettigen Olefinen, zu hohen Umsätzen und zu hohen Selektivitäten zu linearen Oligomerisierungsprodukten.

Das erfindungsgemäße Verfahren zur Herstellung eines Katalysators auf Basis von Nickel-Alumosilicat, welcher ein Silicium/Aluminium-Verhältnis von 8 bis 100, vorzugsweise 20 bis 90, besonders bevorzugt 30 bis 80 und einen Nickelgehalt von 0,5 bis 15 Gew.-% aufweist, umfasst die folgenden Schritte:

a. Herstellen einer Fällungssuspension durch Lösen von mindestens zwei Templaten A und B, mindestens einer Aluminiumquelle, mindestens einer Nickelquelle und mindestens einer Siliciumquelle in einem wasserbasierten Lösemittel, wobei die einzelnen Komponenten so zu wählen sind, dass die hergestellte Suspension einen pH-Wert von mehr als 7,5, vorzugsweise von mehr als 8,5 und besonders bevorzugt von mehr als 9,5 aufweist;

b. Einstellen des pH-Wertes der Fällungssuspension durch Zugabe einer anorganischen Säure auf einen pH-Wert im Bereich von 7,5 bis 12;

c. Fällen der Katalysatorzusammensetzung aus der Fällungssuspension aus Schritt b in einem druckdichten Behälter, vorzugsweise unter einem autogenen Druck von bis zu 15 bar absolut, bei einer Temperatur von 100 bis 180 °C;

d. Abtrennen der in Schritt c ausgefällten Katalysatorzusammensetzung;

e. Trocknen und Kalzinieren der ausgefällten Katalysatorzusammensetzung;

f. Versetzen der kalzinierten Katalysatorzusammensetzung mit einer Lösung, die Ammonium-Ionen enthält;

g. Trocknen und Kalzinieren der mit der Lösung in Schritt f versetzten Katalysatorzusammensetzung zur Herstellung des finalen Katalysators.

In Schritt a, die Herstellung der Fällungssuspension betreffend, werden die mindestens eine Aluminiumquelle, die mindestens eine Nickelquelle und die mindestens eine Siliciumquelle, sowie die mindestens zwei Template A und B in einem wasserbasierten Lösemittel, vorzugsweise Wasser, gelöst. "Lösen" bzw. "gelöst" im Sinne der vorliegenden Erfindung meint nicht nur ein vollständiges Auflösen einer Substanz in dem Lösemittel, sondern umfasst auch das Suspendieren einer Substanz in dem Lösemittel. Alle Komponenten werden entweder gemeinsam gelöst oder zunächst separat im Lösemittel gelöst und anschließend vereinigt. Werden alle Komponenten, insbesondere die mindestens eine Aluminiumquelle, die mindestens eine Nickelquelle und die mindestens eine Siliciumquelle gemeinsam gelöst, können diese vor der Zugabe des Lösemittels vermischt, vorzugsweise gemeinsam gemörsert werden. Während des Lösens der Komponenten in dem Lösemittel kann das Lösemittel mit einer geeigneten Apparatur, beispielsweise einem Magnetrührwerk, gerührt werden. Nachdem die Komponenten im Lösemittel gelöst worden sind, kann die Fällungssuspension für eine gewisse Zeit, vorzugsweise mindestens 5 Minuten weiter gerührt werden. Dabei ist darauf zu achten, dass die einzelnen Komponenten so zu wählen sind, dass die hergestellte Suspension einen pH-Wert von mehr als 7,5, vorzugsweise von mehr als 8,5 und besonders bevorzugt von mehr als 9,5 aufweist. Bevorzugt werden deshalb basisch reagierende Komponenten eingesetzt.

Die Zusammensetzung des resultierenden Oligomerisierungskatalysator wird über die Mengenverhältnisse in der Fällungssuspension eingestellt. Die resultierende Fällungssuspension, die in Schritt a hergestellt wird, enthält vorzugsweise eine Gesamtmenge von Aluminiumquelle, Nickelquelle und Siliciumquelle von 5 bis 30 Gew.-%, vorzugsweise 6 bis 20 Gew.-% und besonders bevorzugt 8 bis 15 Gew.-%. Die Gesamtmenge der Template A und B in der Fällungssuspension kann zwischen 5 und 30 Gew.-%, vorzugsweise 12 bis 25 Gew.-% betragen. Das wasserbasierte Lösungsmittel, vorzugsweise Wasser, kann in einer Menge von 30 bis 80 Gew.-% in der Fällungssuspension vorliegen. Über das molare Verhältnis von Silicium zu Aluminium in der Fällungssuspension, das sich über die Zugabemenge der Silicium- und Aluminiumquelle steuern lässt, kann das Silicium/Aluminium-Verhältnis im fertigen Oligomerisierungskatalysator eingestellt werden. Der Nickelgehalt im Oligomerisierungskatalysator kann über die der Fällungssuspension zuzugebenden Nickelmenge unter der Annahme eingestellt werden, dass die gesamte zugegebene Menge der Aluminiumquelle als $Al_2O_3$ im fertigen Oligomerisierungskatalysator und die gesamte zugegebene Menge der Siliciumquelle als $SiO_2$ im fertigen Oligomerisierungskatalysator vorliegt.

Als Aluminiumquelle können in Schritt a grundsätzlich alle im wasserbasierten Lösemittel löslichen, aber auch nur geringfügig löslichen Aluminiumverbindungen eingesetzt werden. In einer bevorzugten Ausführungsform der vorliegenden Erfindung können als Aluminiumquelle Natriumaluminat, Aluminiumsulfat, Aluminiumnitrat oder Mischungen daraus verwendet werden. Als Nickelquelle können in Schritt a grundsätzlich alle im wasserbasierten Lösemittel löslichen, aber auch nur geringfügig löslichen Nickelverbindungen verwendet werden. In einer bevorzugten Ausführungsform der vorliegenden Erfindung kann die Nickelquelle aus der Gruppe, bestehend aus Nickelnitrat in wasserfreier oder hydratisierter Form, Nickelhalogeniden, wie beispielsweise Nickelchlorid, und Nickelsulfat, ausgewählt werden.

Als Siliciumquelle können in Schritt a grundsätzlich alle im wasserbasierten Lösemittel löslichen, aber auch nur geringfügig löslichen Siliciumverbindungen eingesetzt werden. In einer bevorzugten Ausführungsform der vorliegenden Erfindung kann die Siliciumquelle aus der Gruppe, bestehend aus kolloidalem Siliciumdioxid, Orthosilicaten mit Alkalikationen, wie beispielsweise Natriumorthosilicat oder Natriumtrisilikat, und Tetraalkylorthosilicaten, wie beispielsweise Tetramethylorthosilicat oder Tetraethylorthosilicat, ausgewählt werden. Bevorzugt wird als Siliciumquelle Natriumorthosilicat oder Natriumtrisilikat verwendet.

Die Template A und B sind strukturdirigierenden Verbindungen, die bei der Bildung von mikro- und mesoporösen Strukturen unterstützend wirken sollen. Durch die Verwendung von zwei unterschiedlichen Templaten soll die Ausbildung einer biporösen Struktur bzw. einer bimodalen Porengrößenverteilung angeregt werden.

Das Templat A unterstützt hierbei die Ausbildung einer mikroporösen, möglichst kristallinen Phase. Das Templat A kann aus der Gruppe, bestehend aus primären Aminen mit 2 bis 4 Kohlenstoffatomen, Diaminen mit 2 bis 4 Kohlenstoffatomen, quartären Ammoniumverbindungen mit Alkylgruppen mit 2 bis 4 Kohlenstoffatomen in hydroxidischer oder halogenidischer Form, wie beispielsweise Tetrapropylammoniumhydroxid oder Tetrapropylammoniumbromid, ausgewählt werden. Weiterhin können als Templat A auch Morpholin oder Ammoniumverbindungen in Kombination mit Ethanol wie beispielsweise Ethanolammoniumnitrat verwendet werden. Letztere können auch durch Lösen einer entsprechenden Ammoniumverbindung in einer Ethanol-haltigen wässrigen Lösung in situ gebildet werden. Erfindungsgemäß bevorzugt werden quartäre Ammoniumverbindungen in wässriger Lösung als Templat A verwendet, besonders bevorzugt werden Tetrapropylammoniumhydroxid und Tetrapropylammoniumbromid gelöst in entionisiertem Wasser als Templat A verwendet.

Das Templat B unterstützt die Ausbildung der röntgenamorphen mesoporöse Phase mit im Vergleich zur

kristallinen mikroporösen Phase erhöhtem Porendurchmesser. Als Templat B können Ammoniumverbindungen mit vier Alkylgruppen verwendet werden, wobei mindestens eine der Alkylgruppen eine erhöhte Kettenlänge mit 10 bis 20 Kohlenstoffatomen aufweist. Die verbleibenden Alkylgruppen der Ammoniumverbindungen können Ethyl- bzw. Methylgruppen sein, bevorzugt sind Methylgruppen. Als Anion zum positiv geladenen Tetraalkylammonium-Kation eignen sich insbesondere Halogenid- oder Hydroxidionen. Als Templat B werden erfindungsgemäß vorzugsweise Cetyltrimethylammoniumbromid oder Cetyltrimethylammoniumhydroxid verwendet.

Durch Variation des molaren Verhältnisses von Templat A zu Templat B können die Anteile der mikroporösen und mesoporösen Phase beeinflusst werden. Aus diesem Grund wird ein molares Verhältnis von Templat A zu Templat B im Bereich von 90 : 10 bis 60 : 40 bevorzugt. In einer weiterhin bevorzugten Ausführungsform der vorliegenden Erfindung wird bei der Herstellung des Oligomerisierungskatalysators ein molares Verhältnis von Templat A zu Templat B von 88 : 12 bis 80 : 20 eingesetzt.

Im nachfolgenden Schritt b, das Einstellen des pH-Wertes der Fällungssuspension betreffend, wird zu der Fällungssuspension, vorzugsweise unter Rühren, eine anorganische Säure gegeben, um den pH-Wert auf einen Wert im Bereich von 7,5 bis 12, vorzugsweise von 8,5 bis 11,5, besonders bevorzugt von 9,5 bis 11 einzustellen. Die Zugabe der anorganischen Säure erfolgt bevorzugt schrittweise, beispielsweise durch tröpfchenweise Zugabe. Die anorganische Säure gemäß einer bevorzugten Ausführungsform der vorliegenden Erfindung ausgewählt aus der Gruppe, bestehend aus Schwefelsäure, Salzsäure, Salpetersäure oder einer Mischung daraus. Die Messung des pH-Wertes kann über eine handelsübliche pH-Wertsonde unter ständigem Rühren durchgeführt werden.

Nach dem Einstellen des pH-Wertes in Schritt b wird die Fällungssuspension in einen druckdichten Behälter überführt und die Katalysatorzusammensetzung vorzugsweise unter einem autogenen Druck von bis zu 15 bar absolut in Schritt c ausgefällt. Der Behälter kann beispielsweise ein Autoklav sein. Die innere Wandung des druckdichten Gefäßes kann mit einer inerten Beschichtung versehen sein, um unerwünschte Reaktionen zu vermeiden. Bevorzugt können dafür Einsätze oder Beschichtungen aus Teflon verwendet werden. Das druckdichte Gefäß wird verschlossen und auf eine Temperatur von 100 bis 180 °C, vorzugsweise von 130 bis 170 °C, besonders bevorzugt von 140 bis 160 °C erhitzt. Optional kann die Fällungssuspension währenddessen mit einer geringen Drehgeschwindigkeit gerührt werden, die Rührung sollte jedoch 200 Umdrehungen pro Minute nicht überschreiten. Durch die Erwärmung der Fällungssuspension auf die gewünschte Temperatur kann sich im druckdichten Gefäß ein autogener Druck von bis zu 15 bar absolut einstellen, welcher gehalten werden sollte. Der Druck beträgt vorzugsweise 1 bis 15 bar absolut. Die Temperatur wird vorzugsweise für 10 bis 180 Stunden, weiterhin bevorzugt für 12 bis 72 Stunden, besonders bevorzugt für 24 bis 55 Stunden gehalten. Anschließend wird das druckdichte Gefäß abgekühlt und geöffnet.

Die durch die Fällung in Schritt c erhaltene Katalysatorzusammensetzung wird in Schritt d durch ein geeignetes Verfahren vom verbleibenden Lösemittel abgetrennt. Hierfür eignen sich beispielsweise die Filtration und/oder das Absaugen. Die gefällte Katalysatorzusammensetzung kann anschließend mit einem Überschuss an Wasser, Ethanol oder einer Mischung daraus, vorzugsweise mehrfach gewaschen werden.

In Schritt e wird die gefällte und optional gewaschene Katalysatorzusammensetzung anschließend bei einer Temperatur zwischen 30 und 200°C, vorzugsweise zwischen 40 und 180°C, besonders bevorzugt zwischen 50 und 160 °C getrocknet. Die Dauer der Trocknung wird der Trocknungstemperatur angepasst, wobei die Restfeuchte des Katalysators 20 % nicht überschreiten darf. Die Restfeuchte beschreibt den prozentualen Masseverlust einer Probe, wenn diese auf 110 °C geheizt und solange bei dieser Temperatur gehalten wird, bis keine Masseänderung mehr messbar ist. Diese Messung kann mit dem Moisture Analyzer von der Firma Mettler-Toledo durchgeführt werden. Nach der Trocknung wird die gefällte Katalysatorzusammensetzung in einem geeigneten Ofen kalziniert, unter anderem um die Template oxidativ zu entfernen. Die Kalzination wird vorzugsweise bei einer Temperatur zwischen 400 und 650°C, vorzugsweise zwischen 450 und 600 °C, besonders bevorzugt zwischen 475 und 575°C durchgeführt. Die Kalzination kann unter Verwendung eines Sauerstoff-haltigen Gas, beispielsweise Luft, durchgeführt werden. Die Dauer der Kalzination ist von vielen Faktoren abhängig, beispielsweise von der Templatmenge oder der optional zugeführten Gasmenge. Die Kalzination ist in Abhängigkeit von den genannten Faktoren so lange durchzuführen bis die Template nahezu vollständig (oxidativ) entfernt worden sind.

Nach der Kalzination liegt die in Schritt e getrocknete und kalzinierte Katalysatorzusammensetzung in der Natrium-Form vor. In Schritt f wird die kalzinierte Katalysatorzusammensetzung mit einer Lösung, die Ammonium-Ionen enthält, versetzt, um die Katalysatorzusammensetzung, insbesondere durch Ammoniumionenaustauch, in die Ammonium-Form zu überführen. Die Lösung, die Ammonium-Ionen enthält, ist vorzugsweise eine wässrige Lösung von Ammoniumnitrat, Ammoniumhydroxid oder Ammoniumsulfat, besonders bevorzugt eine wässrige Lösung von Ammoniumnitrat. Die Konzentration der Ammoniumverbindung in der wässrigen Lösung kann im Bereich von 0,01 mol/l bis 1 mol/l, vorzugsweise im Bereich von 0,05 mol/l bis 0,5 mol/l liegen. Schritt f kann bei erhöhter Temperatur, insbesondere bei einer Temperatur zwischen 30 und 90 °C, vorzugsweise zwischen 50 und 80 °C durchgeführt werden.

Als Ammonium-Form wird im Sinne der vorliegenden Erfindung der Zustand verstanden, bei dem man formal von der Existenz einer negativen Ladung am Aluminiumatom ausgeht, an das sich das Ammonium-Kation anlagern kann (siehe rechte Seite der nachfolgenden Abbildung). Bei der Natrium-Form wird die formal negative Ladung am

Aluminiumatom über ein Natrium-Kation maskiert (siehe linke Seite der nachfolgenden Abbildung).

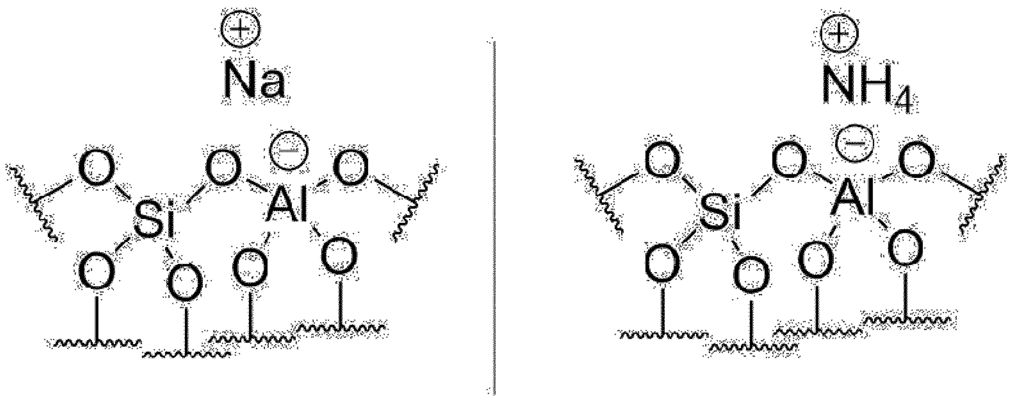

Das Überführen der Katalysatorzusammensetzung in die Ammonium-Form in Schritt f erfolgt in einer bevorzugten Ausführungsform der vorliegenden Erfindung mit der mindestens 5-fachen Menge, vorzugsweise der mindestens 7-fachen Menge, besonders bevorzugt der mindestens 9-fachen Menge an wässriger Lösung, die Ammonium-Ionen enthält. Der molare Ammoniumionenüberschuss bezüglich der Natriumionen beträgt mindestens das 2-fache und maximal das 5-fache, bevorzugt aber das 3-fache. Die Katalysatorzusammensetzung muss nicht nur einmal mit einer Lösung, die Ammonium-Ionen enthält, behandelt werden. Die Lösung kann auch einmal oder mehrfach, insbesondere zweimal ausgetauscht werden. Dafür wird die Katalysatorzusammensetzung von der einen Lösung, die nicht ausgetauschte Ammonium-Ionen enthält, abgetrennt und mit einer frischen Lösung, die die ursprüngliche Menge an Ammonium-Ionen enthält, beaufschlagt.

Die durch den Schritt f erhaltene Katalysatorzusammensetzung kann anschließend durch ein geeignetes Verfahren von der verbleibenden Lösung, die Ammonium-Ionen enthält, abgetrennt werden. Hierfür eignen sich beispielsweise die Filtration und/oder das Absaugen. Die so abgetrennte Katalysatorzusammensetzung kann anschließend mit einem Überschuss an Wasser, Ethanol oder einer Mischung daraus, vorzugsweise mehrfach, gewaschen werden.

Nach Schritt f wird die Katalysatorzusammensetzung in Schritt g erneut getrocknet und kalziniert, um die finale Katalysatorzusammensetzung zu erhalten. Die Katalysatorzusammensetzung kann in Schritt g bei einer Temperatur zwischen 30 und 200°C, vorzugsweise zwischen 40 und 180°C, besonders bevorzugt zwischen 50 und 160°C getrocknet werden. Die Dauer der Trocknung wird der Trocknungstemperatur angepasst, wobei die Restfeuchte des Katalysators 20 % nicht überschreiten darf. Nach der Trocknung wird die Katalysatorzusammensetzung in einem geeigneten Ofen kalziniert. Die Kalzination wird vorzugsweise bei einer Temperatur zwischen 400 und 650°C, vorzugsweise zwischen 450 und 600 °C, besonders bevorzugt zwischen 475 und 575°C durchgeführt. Die Kalzination kann unter Verwendung von Luft, Stickstoff, einem Inertgas, wie Argon, oder Mischungen davon durchgeführt werden.

Nachfolgend kann eine Formgebung der kalzinierten Katalysatorzusammensetzung erforderlich sein, um den Oligomerisierungskatalysator auch industriell einsetzen zu können. Zu den Formgebungsverfahren gehören die dem Fachmann bekannten Verfahren wie Extrudieren, Granulieren, Tablettieren oder Kompaktieren. Als Formgebungsmittel, die zur Katalysatorzusammensetzung hinzugefügt werden können, können z.B. verschiedene Tonmineralien, Kieselsäuren, Schichtsilicate, Tonerden zum Einsatz kommen.

Ein mit diesem Verfahren hergestellter Oligomerisierungskatalysator umfasst das oder besteht aus der Aluminiumquelle, der Nickelquelle und der Siliciumquelle hergestellte(n) Nickel-Alumosilicat mit einer bimodalen Porengrößenverteilung und einem Silicium/Aluminium-Verhältnis von 8 bis 100, vorzugsweise 20 bis 90, besonders bevorzugt 30 bis 80. Der über die Nickelquelle eingestellte Nickelgehalt im Hinblick auf das elementare Nickel beträgt 0,5 bis 15 Gew.-%, vorzugsweise 1 bis 10 Gew.-%, besonders bevorzugt 2 bis 8 Gew.-% bezogen auf die Gesamtzusammensetzung des Oligomerisierungskatalysators.

Der Kohlenstoffgehalt nach der Herstellung des erfindungsgemäßen Oligomerisierungskatalysators, der insbesondere auch Templatreste, die nicht durch die Kalzination entfernt wurden, umfasst, bezeichnet als TOC-Wert (total organic carbon) und mit einem Kohlenstoff-Schwefel-Analysator ermittelt, beträgt 150 bis 1000 Gew.-ppm, vorzugsweise 200 bis 900 Gew.-ppm und besonders bevorzugt 250 bis 800 Gew.-ppm.

Die bimodale Porengrößenverteilung des Oligomerisierungskatalysator ist insbesondere durch eine kristalline, mikroporöse Phase und eine röntgenamorphe, mesoporöse Phase gekennzeichnet, wobei die mikro- und die mesoporöse Phase unterschiedliche mittlere Porendurchmesser aufweisen. Die kristalline mikroporöse Phase des Oligomerisierungskatalysators weist vorzugsweise einen mittleren Porendurchmesser im Bereich von 0,1 bis 2 nm, besonders bevorzugt im Bereich von 0,3 bis 1 nm, bestimmt mittels Stickstoff-Physisorption (Monolayer), auf. Die röntgenamorphe mesoporöse Phase des Oligomerisierungskatalysators verfügt vorzugsweise über einen mittleren Porendurchmesser

im Bereich von 2,5 bis 10 nm, besonders bevorzugt im Bereich von 3 bis 5 nm, bestimmt mittels Quecksilber-Porosimetrie, auf.

Weiterhin bevorzugt weist der erfindungsgemäße Oligomerisierungskatalysator einen DOC (degree of crystallinity) von 3 bis 50%, vorzugsweise 10 bis 47,5%, besonders bevorzugt 20 bis 45%, ermittelt aus röntgenografischen Messungen, auf. Der DOC gibt den Anteil der kristallinen Phase an. Der DOC kann wie folgt bestimmt werden: Es wird zunächst ein Röntgenpulverdiffraktogramm einer Probe des Oligomerisierungskatalysators und ein Referenz-Diffraktogramm einer Probe, die vollständig aus der kristallinen mikroporösen Phase besteht, angefertigt. Für die Auftragung werden die (011)-Reflexe (7,9° 2 Theta) im Diffraktogramm der Probe, die vollständig aus der kristallinen mikroporösen Phase besteht, auf 100 % normiert. Zur Berechnung des DOC wird die Fläche ($A_{Krist}$) der Peaks, die anhand der Referenzpeaks als Peaks der kristallinen Phase identifiziert werden können, durch die Fläche ($A_{gesamt}$) unter der gesamten Signallinie inklusive aller Peaks im Diffraktogramm geteilt und gemäß der nachfolgenden Formel mit 100 % multipliziert:

$$DOC = \frac{A_{Krist}}{A_{gesamt}} \cdot 100\%$$

In einer bevorzugten Ausführungsform der vorliegenden Erfindung weist der Oligomerisierungskatalysator ein Verhältnis des Porenvolumens der kristallinen mikroporösen Phase zum Porenvolumen der röntgenamorphen mesoporösen Phase von 0,6 bis 1,8, vorzugsweise von 0,8 bis 1,6, bestimmt mittels Stickstoff-Physisorption, auf.

Der erfindungsgemäß hergestellte Oligomerisierungskatalysator weist vorzugsweise eine spezifische Oberfläche (berechnet nach BET) von 300 bis 700 $m^2/g$, vorzugsweise 350 bis 600 $m^2/g$, besonders bevorzugt von 400 bis 550 $m^2/g$ auf. Die spezifische Oberfläche wird mittels Stickstoff-Physisorption gemäß DIN-ISO 9277 (Stand: 2014-01) ermittelt.

Der mit dem erfindungsgemäßen Verfahren hergestellte Katalysator kann insbesondere für die Oligomerisierung von C3- bis C6-Olefinen, vorzugsweise C3- bis C5-Olefinen, besonderes bevorzugt von C4-Olefinen oder darauf basierenden olefinhaltigen Einsatzgemischen verwendet werden. Die Olefine oder olefinhaltigen Einsatzgemische werden als Eduktstrom eingesetzt.

Gegenstand der vorliegenden Erfindung ist auch ein Verfahren zur Oligomerisierung von C3- bis C6-Olefinen, wobei ein olefinhaltiges Einsatzgemisch, welches die C3- bis C6-Olefine enthält, in mindestens einer Reaktionszone über einen Katalysator geleitet wird, wobei als Katalysator ein nach den Ansprüchen 1 bis 6 hergestellter Katalysator zur Katalyse der Oligomerisierungsreaktion eingesetzt wird. Eine Reaktionszone umfasst erfindungsgemäß mindestens einen Reaktor und mindestens ein Destillationskolonne, in der die gebildeten Oligomere abgetrennt werden können. Das erfindungsgemäße Verfahren kann auch mit zwei oder mehr Reaktionszonen betrieben werden. Die Oligomerisierung findet vorzugsweise in flüssiger Phase statt.

Als Olefine für das erfindungsgemäße Verfahren werden C3- bis C6-Olefine, bevorzugt C3- bis C5-Olefine, besonders bevorzugt C4-Olefine oder darauf basierende olefinhaltige Einsatzgemische, die auch Anteile an analogen Alkanen enthalten können, eingesetzt. Geeignete Olefine sind unter anderem $\alpha$-Olefine, n-Olefine und Cycloalkene. Bevorzugt sind die als Edukte eingesetzten Olefine n-Olefine. In einer besonders bevorzugten Ausführungsform der vorliegenden Erfindung handelt es sich bei dem Olefin um n-Buten. Der Begriff ‚darauf basierende olefinhaltige Einsatzgemische'ist erfindungsgemäß so zu verstehen, dass er jegliche Art von Gemischen betrifft, die die entsprechenden zu oligomerisierenden Olefine in einer Menge enthalten, die es ermöglicht, die Oligomerisierung durchzuführen. Bevorzugt enthalten die olefinhaltigen Einsatzgemische praktisch keine weiteren ungesättigten Verbindungen und mehrfach ungesättigte Verbindungen wie Diene oder Acetylenderivate. Vorzugsweise werden Olefingemische eingesetzt, die weniger als 5 Gew.-%, insbesondere weniger als 2 Gew.-% an verzweigten Olefinen bezogen auf den Olefinanteil enthalten. Weiterhin bevorzugt werden olefinhaltige Einsatzgemische eingesetzt, die weniger als 2 Gew.-% verzweigte Olefine, insbesondere iso-Olefine, enthalten.

Propylen (C3) wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion oder Extraktionsdestillation des Butadiens oder dessen Selektivhydrierung. In beiden Fällen wird ein praktisch butadienfreier C4-Schnitt erhalten, das Raffinat I. Im zweiten Schritt wird Isobuten aus dem $C_4$-Strom entfernt, z. B. durch Herstellung von Methyl-tert.-butylether (MTBE) durch Umsetzung mit Methanol. Andere Möglichkeiten sind die Umsetzung des Isobutens aus dem Raffinat I mit Wasser zu tert.-Butanol oder die sauer katalysierte Oligomerisierung des Isobutens zu Diisobuten. Der

jetzt isobutenfreie C4-Schnitt, das Raffinat II, enthält wie gewünscht die linearen Butene und gegebenenfalls Butane. Optional kann noch das 1-Buten destillativ abgetrennt werden. Beide Fraktionen, die mit But-1-en oder die mit But-2-en, können im erfindungsgemäßen Verfahren eingesetzt werden.

In einer weiteren bevorzugten Ausführungsform werden C4-olefinhaltige Stoffströme dem Verfahren als olefinhaltige Einsatzgemische zugeführt. Geeignete olefinhaltige Einsatzgemische sind dafür unter anderem das Raffinat I (butadienfreier C4-Schnitt des Steamcrackers) sowie das Raffinat II (butadien- und isobutenfreier C4-Schnitt des Steamcrackers).

Eine weitere Möglichkeit, geeignete olefinhaltige Einsatzgemische herzustellen, besteht darin, Raffinat I, Raffinat II oder ein ähnlich zusammengesetztes Kohlenwasserstoffgemisch in einer Reaktivkolonne zu hydroisomerisieren. Dabei kann u. a. ein Gemisch gewonnen werden, das aus 2-Butenen, geringen Anteilen 1-Buten und gegebenenfalls n-Butan sowie Isobutan und Isobuten besteht.

Die Oligomerisierung erfolgt in der Regel bei einer Temperatur im Bereich von 50 bis 200 °C, vorzugsweise 60 bis 180 °C, bevorzugt im Bereich von 60 bis 130°C, und bei einem Druck von 10 bis 70 bar, bevorzugt von 20 bis 55 bar. Sofern die Oligomerisierung in flüssiger Phase erfolgen soll, müssen die Parameter Druck und Temperatur hierfür so gewählt werden, dass der Eduktstrom (die eingesetzten Olefine oder olefinhaltigen Einsatzgemische) in flüssiger Phase vorliegt. Die gewichtbasierten Raumgeschwindigkeiten (Reaktandmasse pro Katalysator pro Zeit; weight hourly space velocity (WHSV)) des olefinhaltigen Einsatzgemisches befinden sich im Bereich zwischen 1 g Reaktand pro g Katalysator und pro h (= 1 $h^{-1}$) und 190 $h^{-1}$, vorzugsweise zwischen 2 $h^{-1}$ und 35 $h^{-1}$, besonders bevorzugt zwischen 3 $h^{-1}$ und 25 $h^{-1}$.

In einer Ausführungsform der vorliegenden Erfindung beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den Iso-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum Iso-Index einer C8-Fraktion bei. Je niedriger der Iso-Index ist, umso linearer sind die Moleküle in der jeweiligen Fraktion aufgebaut. Der Iso-Index errechnet sich nach folgender allgemeiner Formel:

$$\frac{(\text{einfach verzweigte Dimere (Gew.-\%)} + 2 \text{ x zweifach verzweigte Dimere (Gew.-\%)})}{100}$$

Demnach besitzt ein Dimerengemisch mit einem Iso-Index von 1,0 im Mittel genau eine Methylverzweigung pro Dimerenmolekül.

Der Iso-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein $C_9$-Alkoholgemisch. Das C9-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH.

Auch ohne weitere Ausführungen wird davon ausgegangen, dass ein Fachmann die obige Beschreibung im weitesten Umfang nutzen kann. Die bevorzugten Ausführungsformen der vorliegenden Erfindung und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

Beispiele:

Katalysatorsynthese (erfindungsgemäßer Katalysator 1):

Bei der Synthese wurden Cetyltrimethylammoniumbromid, Tetrapropylammoniumbromid, Natriumaluminat, Nickelnitrathexahydrat und Natriumtrisilikat in entionisiertem Wasser gelöst und gerührt bis eine milchig-weiße, leicht grünliche Lösung gebildet hat. Das Natriumtrisilikat wurde bezogen auf ein Si-Atom im Vergleich zum Natriumaluminat in 40-fachem molaren Überschuss zugegeben. Die Lösung wurde durch Hinzutropfen von Schwefelsäure auf einen pH-Wert von etwa 10 eingestellt. Anschließend wurde die Lösung in einen Autoklav mit Tefloneinsatz gegeben und auf 130 bis 170 °C erhitzt, wodurch die Katalysatorzusammensetzung ausgefällt wird. Nach der Fällung wurde der gefällte

Katalysator abfiltriert und mit Wasser und Ethanol gewaschen. Zur Entfernung der Template wurde die Katalysatorzusammensetzung zunächst getrocknet und dann bei > 500 °C kalziniert. Im Anschluss wurde die Katalysatorzusammensetzung mindestens zweimal mit einer Ammoniumnitratlösung bei > 50 °C behandelt. Danach erfolgte eine weitere Trocknung und die finale Kalzination bei > 400 °C.

Der so hergestellt Katalysator wies einen Nickelgehalt von 3 Gew.-% und ein Si/Al-Verhältnis von 40 auf.

Katalysatorsynthese (erfindungsgemäßer Katalysator 2):

Die Synthese wurde wie für den Katalysator 1 durchgeführt mit dem Unterschied, dass das Natriumtrisilikat in 10-facher Menge zugegeben wurde. Der so hergestellt Katalysator wies einen Nickelgehalt von 3 Gew.-% und ein Si/Al-Verhältnis von 10 auf.

Katalysatorsynthese (erfindungsgemäßer Katalysator 3):

Die Synthese wurde wie für den Katalysator 1 durchgeführt mit dem Unterschied, dass das Natriumtrisilikat in 70-facher Menge zugegeben wurde. Der so hergestellt Katalysator wies einen Nickelgehalt von 3 Gew.-% und ein Si/Al-Verhältnis von 70 auf.

Katalysatorsynthese (nicht erfindungsgemäßer Katalysator 4):

Die Synthese wurde wie für den Katalysator 1 durchgeführt mit dem Unterschied, dass kein Natriumaluminat zugegeben wurde. Der so hergestellt Katalysator wies einen Nickelgehalt von 3 Gew.-% auf. Ein Si/Al-Verhältnis kann nicht bestimmt werden, da kein Al vorliegt.

Einsatz der Katalysatoren in der Oligomerisierung:

Es wurden jeweils ca. 12 g des Katalysators in ein Metallrohr mit einem Innendurchmesser vom 6 mm eingefüllt. Vor und hinter dem Katalysator wurden Glasperlen mit einem Durchmesser von 2 mm gegeben, die als Vorheiz- bzw. Abkühlphase dienen. Die Oligomerisierung wurde unter Verwendung von zwei unterschiedlichen Beschickungsströmen bei 30 bar und einer Belastung von 7,5 g/h Buten pro Gram Katalysator durchgeführt, wobei die Reaktionstemperatur zwischen 80 °C und 100 °C variiert wurde. Die Produkte wurden gaschromatographisch auf den Umsatz an Butenen und die Linearität der Oktene analysiert. Die Zusammensetzungen des Beschickungsstroms für die Oligomerisierung zeigt die nachfolgende Tabelle 1.

Die für den Beschickungsstrom in Abhängigkeit von der Temperatur erzielten Umsätze und Selektivitäten für die Katalysatoren 1 bis 3 (erfindungsgemäß) und Katalysator 4 (nicht erfindungsgemäß), sowie die daraus resultierenden ISO-Indices sind in den Tabellen 2 bis 5 angegeben.

Tabelle 1: Zusammensetzung des Beschickungsstroms

| | Beschickungsstrom 1 |
|---|---|
| iso-Butan | 8,0 % |
| n-Butan | 15,3 % |
| trans-2-Buten | 27,9 % |
| 1-Buten | 32,7 % |
| iso-Buten | 0,9 % |
| cis-2-Buten | 15,2 % |

Tabelle 2: Umsatz und Iso-Index bei der Oligomerisierung unter Verwendung von Katalysator 1

| Belastung (Zulauf C4-Olefine in g/h pro Masse Katalysator in g) als WSHV : 7,5 $h^{-1}$ | | | |
|---|---|---|---|
| | Temperatur | Umsatz | Iso-Index |
| | | bezogen auf C4-Oelfine | |
| Katalysator 1 (erfindungsgemäß) | 80 °C | 7,8% | 0,92 |

Tabelle 3: Umsatz und Iso-Index bei der Oligomerisierung unter Verwendung von Katalysator 2

| Belastung (Zulauf C4-Olefine in g/h pro Masse Katalysator in g) als WSHV : 7,5 $h^{-1}$ | | | |
|---|---|---|---|
| | Temperatur | Umsatz | Iso-Index |
| | | bezogen auf C4-Oelfine | |
| Katalysator 2 (erfindungsgemäß) | 80 °C | 3,0% | 0,90 |

Tabelle 4: Umsatz und Iso-Index bei der Oligomerisierung unter Verwendung von Katalysator 3

| Belastung (Zulauf C4-Olefine in g/h pro Masse Katalysator in g) als WSHV : 7,5 $h^{-1}$ | | | |
|---|---|---|---|
| | Temperatur | Umsatz | Iso-Index |
| | | bezogen auf C4-Oelfine | |
| Katalysator 3 (erfindungsgemäß) | 80 °C | 9,1% | 0,87 |

Tabelle 5: Umsatz und Iso-Index bei der Oligomerisierung unter Verwendung von Katalysator 4

| Belastung (Zulauf C4-Olefine in g/h pro Masse Katalysator in g) als WSHV : 7,5 $h^{-1}$ | | | |
|---|---|---|---|
| | Temperatur | Umsatz | Iso-Index |
| | | bezogen auf C4-Oelfine | |
| Katalysator 4 (nicht erfindungsgemäß) | 80 °C | 2,2% | 0,87 |

Im Gegensatz zum nicht erfindungsgemäßen Katalysator 4 zeigt sich überraschenderweise, dass sich mit den erfindungsgemäßen Katalysatoren 1 bis 3 bei ähnlichen Iso-Indices eine erkennbare Steigerung des Umsatzes erreichen lässt.

## Patentansprüche

**1.** Verfahren zur Herstellung eines Katalysators auf Basis von Nickel-Alumosilicat, welcher ein Silicium/Aluminium-Verhältnis von 8 bis 100 und einen Nickelgehalt von 0,5 bis 15 Gew.-% aufweist, wobei das Verfahren die folgenden Schritte umfasst:

a. Herstellen einer Fällungssuspension durch Lösen von mindestens zwei Templaten A und B, mindestens einer Aluminiumquelle, mindestens einer Nickelquelle und mindestens einer Siliciumquelle in einem wasserbasierten Lösemittel, vorzugsweise Wasser, wobei die einzelnen Komponenten so zu wählen sind, dass die hergestellte Suspension einen pH-Wert von mehr als 7,5 aufweist;
b. Einstellen des pH-Wertes durch Zugabe einer anorganischen Säure auf einen pH-Wert im Bereich von 7,5 bis 12;
c. Fällen der Katalysatorzusammensetzung aus der Fällungssuspension aus Schritt b in einem druckdichten Behälter bei einer Temperatur von 100 bis 180 °C;
d. Abtrennen der in Schritt c ausgefällten Katalysatorzusammensetzung;
e. Trocknen und Kalzinieren der ausgefällten Katalysatorzusammensetzung;
f. Versetzen der kalzinierten Katalysatorzusammensetzung mit einer Lösung, die Ammonium-Ionen enthält;

g. Trocknen und Kalzinieren der mit der Lösung in Schritt f versetzten Katalysatorzusammensetzung zur Herstellung des finalen Katalysators.

2. Verfahren nach Anspruch 1, wobei die Aluminiumquelle aus der Gruppe, bestehend aus Natriumaluminat, Aluminiumsulfat, Aluminiumnitrat und Mischungen daraus, ausgewählt wird.

3. Verfahren nach Anspruch 1 oder 2, wobei die Nickelquelle aus der Gruppe, bestehend aus Nickelnitrat in wasserfreier oder hydratisierter Form, Nickelhalogeniden, wie Nickelchlorid, und Nickelsulfat, ausgewählt wird.

4. Verfahren nach einem der Ansprüche 1 bis 3, wobei die Siliciumquelle aus der Gruppe, bestehend aus kolloidalem Siliciumdioxid, Orthosilicaten mit Alkalikationen, wie beispielsweise Natriumorthosilicat oder Natriumtrisilikat, und Tetraalkylorthosilicaten, wie beispielsweise Tetramethylorthosilicat oder Tetraethylorthosilicat, ausgewählt wird.

5. Verfahren nach einem der Ansprüche 1 bis 4, wobei das Templat A aus der Gruppe, bestehend aus primären Aminen mit 2 bis 4 Kohlenstoffatomen, Diaminen mit 2 bis 4 Kohlenstoffatomen, quartären Ammoniumverbindungen mit Alkylgruppen mit 2 bis 4 Kohlenstoffatomen in hydroxidischer oder halogenidischer Form, wie beispielsweise Tetrapropylammoniumhydroxid oder Tetrapropylammoniumbromid, ausgewählt wird.

6. Verfahren nach einem der Ansprüche 1 bis 5, wobei als Templat B eine Ammoniumverbindung mit vier Alkylgruppen verwendet wird, wobei mindestens eine der Alkylgruppen eine erhöhte Kettenlänge mit 10 bis 20 Kohlenstoffatomen aufweist.

7. Verfahren nach einem der Ansprüche 1 bis 6, wobei der Katalysator ein Silicium/Aluminium-Verhältnis von 20 bis 90 aufweist.

8. Verfahren nach einem der Ansprüche 1 bis 6, wobei der Katalysator ein Silicium/Aluminium-Verhältnis von 30 bis 80 aufweist.

9. Verfahren nach einem der Ansprüche 1 bis 8, wobei die in Schritt a hergestellte Fällungssuspension eine Gesamtmenge von Aluminiumquelle, Nickelquelle und Siliciumquelle von 5 bis 30 Gew.-% enthält.

10. Verfahren nach einem der Ansprüche 1 bis 9, wobei die Gesamtmenge der Template A und B in der in Schritt a hergestellten Fällungssuspension kann zwischen 5 und 30 Gew.-% beträgt.

11. Verfahren nach einem der Ansprüche 1 bis 10, wobei die in Schritt a hergestellte Fällungssuspension das wasserbasierte Lösungsmittel in einer Menge von 30 bis 80 Gew.-% enthält.

12. Verfahren nach einem der Ansprüche 1 bis 11, wobei das Fällen der Katalysatorzusammensetzung aus der Fällungssuspension in Schritt c unter einem autogenen Druck von bis zu 15 bar absolut erfolgt.

13. Verwendung des nach den Ansprüchen 1 bis 6 hergestellten Katalysators zur Katalyse einer Olefinoligomerisierungsreaktion.

14. Verfahren zur Oligomerisierung von C3- bis C6-Olefinen, wobei ein olefinhaltiges Einsatzgemisch, welches die C3- bis C6-Olefine enthält, in mindestens einer Reaktionszone über einen Katalysator geleitet wird, wobei als Katalysator ein nach den Ansprüchen 1 bis 6 hergestellter Katalysator zur Katalyse der Oligomerisierungsreaktion eingesetzt wird.

Europäisches Patentamt
European Patent Office
Office européen des brevets

# EUROPÄISCHER RECHERCHENBERICHT

Nummer der Anmeldung
EP 19 16 2180

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 260 501 A (BHORE NAZEER A [US] ET AL) 9. November 1993 (1993-11-09)<br>* Zusammenfassung *<br>* Spalte 4, Zeilen 41-56 *<br>* Spalte 7, Zeilen 31-43 *<br>* Spalte 8, Zeile 5 - Spalte 9, Zeile 41 *<br>* Beispiele 1A, 1B, 2 *<br>----- | 1-14 | INV.<br>B01J29/04<br>B01J37/08<br>B01J37/03<br>B01J37/02<br>B01J35/00<br>B01J35/10<br>C01B39/04<br>C07C2/10<br><br>ADD.<br>B01J21/12<br>B01J23/755 |
| X | US 5 134 242 A (LE QUANG N [US] ET AL) 28. Juli 1992 (1992-07-28)<br>* Zusammenfassung *<br>* Spalte 9, Zeilen 3-17 *<br>* Spalte 10, Zeile 33 - Spalte 11, Zeile 8 *<br>* Spalte 12, Zeile 67 - Spalte 13, Zeile 46 *<br>* Beispiele 3,6,11 *<br>----- | 1-14 | |
| A | MOUSSA SARA ET AL: "Heterogeneous oligomerization of ethylene to liquids on bifunctional Ni-based catalysts: The influence of support properties on nickel speciation and catalytic performance", CATALYSIS TODAY, ELSEVIER, AMSTERDAM, NL, Bd. 277, 11. Januar 2016 (2016-01-11), Seiten 78-88, XP029743467, ISSN: 0920-5861, DOI: 10.1016/J.CATTOD.2015.11.032<br>* Zusammenfassung *<br>* Seiten 80-82 *<br>----- | 1-14 | RECHERCHIERTE SACHGEBIETE (IPC)<br>B01J<br>C07C |

Der vorliegende Recherchenbericht wurde für alle Patentansprüche erstellt

1

| Recherchenort | Abschlußdatum der Recherche | Prüfer |
|---|---|---|
| München | 20. August 2019 | Goebel, Matthias |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 19 16 2180

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-08-2019

| Im Recherchenbericht angeführtes Patentdokument | Datum der Veröffentlichung | Mitglied(er) der Patentfamilie | Datum der Veröffentlichung |
|---|---|---|---|
| US 5260501 A | 09-11-1993 | KEINE | |
| US 5134242 A | 28-07-1992 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9514647 A1